Europäisches Patentamt

European Patent Office

Office européen des brevets

(11) Veröffentlichungsnummer: **0 512 213 B1**

(12) **EUROPÄISCHE PATENTSCHRIFT**

(45) Veröffentlichungstag der Patentschrift: **21.06.95**

(21) Anmeldenummer: **92103834.5**

(22) Anmeldetag: **06.03.92**

(51) Int. Cl.⁶: **C08G 18/79**, C08G 18/80, C07D 251/34, C09D 175/04

(54) Verfahren zur Herstellung eines blockierten Lackpolyisocyanats.

(30) Priorität: **10.05.91 DE 4115402**

(43) Veröffentlichungstag der Anmeldung:
**11.11.92 Patentblatt 92/46**

(45) Bekanntmachung des Hinweises auf die Patenterteilung:
**21.06.95 Patentblatt 95/25**

(84) Benannte Vertragsstaaten:
**BE CH DE FR GB IT LI NL SE**

(56) Entgegenhaltungen:
**EP-A- 0 010 589**
**DE-A- 2 631 733**
**DE-A- 2 712 931**

**WORLD PATENTS INDEX LATEST Week 9021, Derwent Publications Ltd., London, GB; AN90-159083**

(73) Patentinhaber: **HÜLS AKTIENGESELLSCHAFT**

**D-45764 Marl (DE)**

(72) Erfinder: **Gras, Rainer, Dr.**
**Im Ostholz 49 a**
**W-4630 Bochum 5 (DE)**
Erfinder: **Wolf, Elmar, Dr.**
**Stauffenbergstrasse 7**
**W-4350 Recklinghausen (DE)**

**Beschreibung**

Die vorliegende Erfindung betrifft ein Verfahren zur Herstellung eines Lackpolyisocyanats auf Basis eines ε-Caprolactam blockierten, Isocyanuratgruppen enthaltenden 1-Isocyanato-3.5.5-trimethyl-5-isocyanatomethyl-cyclohexans (Isophorondiisocyanat, IPDI).

Bei den PUR-Pulverlacken, die erstmals 1970 in der Literatur beschrieben wurden, nehmen die in der DE-PS 27 35 497 vorgestellten PUR-Pulver infolge ihrer überlegenen Witterungs- und Wärmefarbstabilität eine herausragende Stellung ein.

Die Herstellung der Härterkomponente dieser PUR-Pulver wird in der DE-PS 27 12 931 beschrieben. Sie erfolgt in 2 Stufen, wobei in der 1. Stufe IPDI trimerisiert wird, und nach erfolgter Trimerisierung dann in der 2. Stufe die ε-Caprolactam-Blockierung durchgeführt wird. Nachteilig bei dieser Herstellung ist einmal die lange Reaktionszeit bei hoher Temperatur, die zur Verfärbung im Reaktionsprodukt führen kann. So beträgt die Reaktionszeit je nach Isocyanuratgehalt des trimerisierten IPDI 0,5 - 3 h. Zum anderen muß zur Desaktivierung des Trimerisierungskatalysators entweder Vakuum angelegt, oder mit Stickstoff gestrippt werden; d. h., daß zur Herstellung des trimerisierten IPDI neben dem Reaktor noch zusätzliche Geräte (Vakuum, Kühlfalle) erforderlich sind. Wird ein nach DE-PS 27 12 931 hergestelltes, Isocyanuratgruppen enthaltendes IPDI mit dem ε-Caprolactam blockiert, so riecht das blockierte IPDI-Isocyanurat-Gemisch intensiv nach Amin. Dieser unangenehme Geruch des Pulverhärters, der vor allem beim Mahlen auftritt, kann beseitigt werden, wenn zur ε-Caprolactam-Blockierung ein trimerisiertes IPDI eingesetzt wird, das nach beendeter Trimerisierung, d. h. also nach Erreichen des gewünschten Isocyanuratgehalts unter Anlegen von Vakuum, ca. 0,5 h auf ca. 180 °C erhitzt und der Katalysator damit desaktiviert wird. Dies bedeutet einen zusätzlichen Verfahrensschritt.

Aufgabe der vorliegenden Erfindung war es deshalb, ein wenig aufwendiges Verfahren zur Herstellung eines geruchsfreien Lackpolyisocyanats auf Basis eines ε-Caprolactam blockierten, Isocyanuratgruppen enthaltenden IPDI zu finden, das die Nachteile der bis jetzt bekannten Herstellungsverfahren nicht besitzt.

Diese Aufgabe konnte überraschenderweise dadurch gelöst werden, daß IPDI in der 1. Stufe in an sich bekannter Weise mit N-(Hydroxyalkyl)-ammoniumsalzen von Carbonsäuren trimerisiert, nach erfolgter Trimerisierung mit 0,1 - 1 Gew.-% $H_3PO_3$ oder $H_3PO_4$ umgesetzt wird, und in der 2. Stufe die ε-Caprolactam-Blockierung wiederum in an sich bekannter Weise erfolgt.

Gegenstand der Erfindung ist ein Verfahren zur Herstellung eines Lackpolyisocyanats auf Basis eines ε-Caprolactam blockierten, Isocyanuratgruppen enthaltenden Isophorondiisocyanats, das in an sich bekannter Weise durch Trimerisierung des Isophorondiisocyanats mit quaternären N-(Hydroxyalkyl)-ammoniumsalzen von Carbonsäuren und anschließende ε-Caprolactam-Blockierung des Isocyanatocyanurat-Gemisches hergestellt wird, welches dadurch gekennzeichnet ist, daß vor der ε-Caprolactam-Blockierung das Isocyanatoisocyanurat-Gemisch des IPDI mit 0,1 - 3 Gew.-% Phosphor- bzw. phosphoriger Säure versetzt wird und pro NCO-Äquivalent des Isocyanatoisocyanurat-Gemisches 0,7 - 1 mol ε-Caprolactam zur Reaktion gelangen.

Durch die Zugabe von Phosphor- bzw. phosphoriger Säure konnte auf die aufwendige Zerstörung des Katalysators verzichtet werden. Obendrein ist die $H_3PO_4$- bzw. $H_3PO_3$-Zugabe mit einer deutlichen Farbaufhellung des Reaktionsprodukts verbunden.

Die quaternären Ammoniumsalze werden in den DE-PSS 26 31 733 und 29 16 201 zur Trimerisierung von organischen Isocyanaten beschrieben und sind nicht Gegenstand der vorliegenden Erfindung.

Zur Trimerisierung des IPDI (in Charge) im vorliegenden Verfahren wird der Katalysator in Mengen von 0,05 - 0,5 Gew.-% (bezogen auf IPDI) unter intensiver Rührung dem IPDI bei Raumtemperatur bis 80 °C zugegeben. Der Umsatz, d. h. die Konzentration an Isocyanuratgruppen, kann durch die Katalysatorkonzentration wie auch Reaktionstemperatur beliebig eingestellt werden. Wird der Katalysator bei erhöhter Temperatur, z. B. 80 °C, dem IPDI zugegeben, so steigt die Temperatur des Reaktionsgemisches innerhalb weniger Minuten je nach Katalysatorkonzentration auf 110 bis 170 °C. Nach Erreichen des Temperaturmaximums ist die Reaktion beendet. Das Temperaturmaximum ist indirekt ein Maß für den Umsetzungsgrad. Je höher das Temperaturmaximum, desto größer ist auch der Umsatz. Man kann aber auch nach folgender Verfahrensvariante trimerisieren:

IPDI wird mit 0,05 - 0,5 Gew.-% Katalysator (quaternäres Ammoniumsalz) bei Raumtemperatur unter intensiver Rührung versetzt und zügig auf 60 - 70 °C erhitzt. In diesem Temperaturbereich beginnt die Umsetzung. Durch Zudosieren von Frisch-IPDI wird die Temperatur des Reaktionsgemisches so eingestellt, daß sie 80 °C nicht übersteigt. Fällt die Temperatur des Reaktionsgemisches, so wird zur Vervollständigung der Reaktion noch ca. 0,5 h bei 85 °C weitererhitzt. Anschließend erfolgt die Zugabe von 0,1 - 1 Gew.-% $H_3PO_4$ bzw. $H_3PO_3$. Für das erfindungsgemäße Verahren ist es zwingend notwendig, daß die $H_3PO_4$- bzw. $H_3PO_3$-Zugabe nach beendeter Trimerisierung erfolgt. Wird sie vor bzw. während der Trimerisierung zugegeben, wird der Umsatz ganz bzw. stark reduziert. Für jeden Katalysator ist eine

bestimmte Mindestmenge an $H_3PO_4$ bzw. $H_3PO_3$ erforderlich. Wird diese unterschritten, so zeigt das Reaktionsprodukt die eingangs erwähnten Nachteile auf (penetranter Amingeruch beim Verarbeiten).

Nach beendeter $H_3PO_4$- bzw. $H_3PO_3$-Zugabe wird das Reaktionsgemisch auf 120 °C erhitzt. Bei dieser Temperatur erfolgt in bekannter Weise die $\epsilon$-Caprolactamzugabe, wobei in der Regel auf 1 NCO-Äquivalent des Polyisocyanatoisocyanurat-Gemisches 0,7 - 1 mol, bevorzugt 1 mol $\epsilon$-Caprolactam zur Reaktion kommen. Mit den erfindungsgemäß hergestellten Verfahrensprodukten lassen sich in Verbindung mit der Polyol-Komponente überbrennbare, witterungsbeständige PUR-Lacke, insbesondere Pulverlacke, herstellen.

Die gemäß Anspruch 1 hergestellten Lackpolyisocyanate werden für PUR-Pulver und PUR-Einbrennlacke verwendet.

Als Reaktionspartner für PUR-Pulverlacke kommen Verbindungen infrage, welche solche funktionellen Gruppen tragen, die sich mit Isocyanatgruppen während des Härtungsprozesses in Abhängigkeit von der Temperatur und Zeit umsetzen, z. B. Hydroxyl-, Carboxyl-, Mercapto-, Amino-, Urethan- und (Thio)-Harnstoffgruppen. Als Polymere können Polymerisate, Polykondensate und Polyadditionsverbindungen eingesetzt werden.

Bevorzugte Komponenten sind in erster Linie Polyether, Polythioether, Polyacetale, Polyesteramide, Epoxidharze mit Hydroxylgruppen im Molekül, Aminoplaste und ihre Modifizierungsprodukte mit polyfunktionellen Alkoholen, Polyazomethine, Polyurethane, Polysulfonamide, Melaminabkömmlinge, Celluloseester und -ether, teilweise verseifte Homo- und Copolymerisate von Vinylestern, insbesondere aber Polyester und Acrylatharze.

Die einzusetzenden hydroxylgruppenhaltigen Polyester haben eine OH-Funktionalität von 2,5 bis 5, bevorzugt von 3 bis 4,2, ein mittleres Molekulargewicht von 1 800 bis 5 000, bevorzugt von 2 300 bis 4 500, eine OH-Zahl von 25 bis 120 mg KOH/g, bevorzugt von 30 bis 90 mg KOH/g, eine Viskosität von < 80 000 mPa•s, bevorzugt von < 60 000 mPa•s, besonders bevorzugt von < 40 000 mPa•s bei 160 °C und einem Schmelzpunkt von ≥ 70 °C bis ≤ 120 °C, bevorzugt von ≥ 75 °C bis ≤ 100 °C.

Für die Herstellung von Polyestern bevorzugte Carbonsäuren können aliphatischer, cycloaliphatischer, aromatischer und/oder heterocyclischer Natur sein und gegebenenfalls durch Halogenatome substituiert und/oder ungesättigt sein. Als Beispiele hierfür seien genannt:

Bernstein-, Adipin-, Kork-, Azelain, Sebacin-, Phthal-, Terephthal-, Isophthal-, Trimellith-, Pyromellith-, Tetrahydrophthal-, Hexahydrophthal-, Hexahydroterephthal-, Di- und Tetrachlorphthal-, Endomethylentetrahydrophthal-, Glutar-, Malein- und Fumarsäure bzw. - soweit zugänglich - deren Anhydride, Terephthalsäuredimethylester, Terephthalsäure-bis-glykolester, weiterhin cyclische Monocarbonsäuren wie Benzoesäure, p-tert.-Butylbenzoesäure oder Hexahydrobenzoesäure.

Als mehrwertige Alkohole kommen z. B. Ethylenglykol, Propylenglykol-1.2 und -1.3, Butylenglykol-1.4 und -2.3, Di-$\beta$-hydroxyethylbutandiol, Hexandiol-1.6, Octandiol-1.8, Neopentylglykol, Cyclohexandiol, Bis-(1.4-hydroxymethyl)-cyclohexan, 2.2-Bis-(4-hydroxycyclohexyl)-propan, 2.2-Bis-{4($\beta$-hydroxyethoxy)-phenyl}-propan, 2-Methyl-propandiol-1.3, 3-Methyl-pentandiol-1.5, 2.2.4(2.4.4)-Trimethylhexandiol-1.6, Glycerin, Trimethylolpropan, Trimethylolethan, Hexantriol-1.2.6, Butantriol-1.2.4, Tris($\beta$-hydroxyethyl)-isocyanurat, Pentaerythrit, Mannit und Sorbit sowie Diethylenglykol, Triethylenglykol, Tetraethylenglykol, Dipropylenglykol, Polypropylenglykole, Polybutylenglykole, Xylylenglykol und Hydroxypivalinsäureneopentylglykolester, infrage.

Auch Mono- und Polyester aus Lactonen, z. B. $\epsilon$-Caprolacton oder Hydroxycarbonsäuren, z. B. Hydroxypivalinsäure, Ω-Hydroxydecansäure, Ω-Hydroxycapronsäure, Thioglykolsäure, können eingesetzt werden. Polyester aus den obengenannten Polycarbonsäuren bzw. deren Derivaten und Polyphenolen, wie Hydrochinon, Bisphenol-A, 4.4'-Dihydroxybiphenyl oder Bis-(4-hydroxyphenyl)-sulfon; Polyester der Kohlensäure, die aus Hydrochinon, Diphenylolpropan, p-Xylylenglykol, Ethylenglykol, Butandiol oder Hexandiol-1.6 und anderen Polyolen durch übliche Kondensationsreaktionen, z. B. mit Phosgen oder Diethyl- bzw. Diphenylcarbonat, oder aus cyclischen Carbonaten wie Glykolcarbonat oder Vinylidencarbonat, durch Polymerisation in bekannter Weise erhältlich sind; Polyester der Kieselsäure, Polyester der Phosphorsäure, z. B. aus Methan-, Ethan-, $\beta$-Chlorethan-, Benzol- oder Styrolphosphorsäure-, -phosphorsäurechlorid oder -phosphorsäureester und Polyalkoholen oder Polyphenolen der obengenannten Art; Polyester der Borsäure; Polysiloxane, wie z. B. die durch Hydrolyse von Dialkyldichlorsilanen mit Wasser und nachfolgende Behandlung mit Polyalkoholen, die durch Anlagerung von Polysiloxan-dihydriden an Olefine, wie Alkylalkohol oder Acrylsäure, erhältlichen Produkte.

Bevorzugte Polyester sind z. B. auch die Reaktionsprodukte von Polycarbonsäuren und Glycidylverbindungen, wie sie z. B. in der DE-OS 24 10 513 beschrieben sind.

Beispiele für Glycidylverbindungen, die verwendet werden können, sind Ester des 2.3-Epoxy-1-propanols mit monobasischen Säuren, die 4 bis 18 Kohlenstoffatome haben, wie Glycidylpalmitat, Glycidyllaurat und Glycidylstearat, Alkylenoxide mit 4 bis 18 Kohlenstoffatomen, wie Butylenoxid und Glycidylether, wie Octylglycidylether.

Als Dicarbonsäuren können bei diesem Verfahren sämtliche unter II im folgenden aufgeführte Polycarbonsäuren verwendet werden, Monocarbonsäuren, welche beispielsweise unter III aufgeführt sind, können ebenfalls eingesetzt werden.

Bevorzugte Komponenten sind auch monomere Ester, z. B. Dicarbonsäure- bis-(hydroxy(alkohol)ester, Monocarbonsäureester von mehr als zweiwertigen Polyolen und Oligoester, die durch Kondensationsreaktionen aus in der Lackchemie üblichen Rohstoffen hergestellt werden können. Als solche sind z. B. anzusehen:

I. Alkohole mit 2 bis 24, vorzugsweise 2 bis 10 C-Atomen, und 2 bis 6 an nicht-aromatischen C-Atomen gebundenen OH-Gruppen, z. B. Ethylenglykol, Propylenglykol, Diethylenglykol, Dipropylenglykol, Butandiole, Neopentylglykol, Hexandiole, Hexantriole, Perhydrobisphenol, Dimethylolcyclohexan, Glycerin, Trimethylolethan, Trimethylolpropan, Pentaerythrit, Dipentaerythrit, Mannit.

II. Di- und Polycarbonsäuren mit 4 bis 36 C-Atomen und 2 bis 4 Carboxylgruppen sowie deren veresterungsfähige Derivate, wie Anhydride und Ester, z. B. Phthalsäure(anhydrid), Isophthalsäure, Terephthalsäure, Alkyltetrahydrophthalsäure, Endomethylentetrahydrophthalsäureanhydrid, Adipinsäure, Bernsteinsäure, Maleinsäure, Fumarsäure, Dimerfettsäuren, Trimellithsäure, Pyromellithsäure, Azelainsäure;

III. Monocarbonsäuren mit 6 bis 24 C-Atomen, z. B. Caprylsäure, 2-Ethylhexansäure, Benzoesäure, p-tert.-Butylbenzoesäure, Hexahydrobenzoesäure, Monocarbonsäuregemische natürlicher Öle und Fette, wie Cocosölfettsäure, Sojaölfettsäure, Ricinenfettsäure, hydrierte und isomerisierte Fettsäuren, wie "Konjuvandol"-Fettsäure sowie deren Gemische, wobei die Fettsäuren auch als Glyceride einsetzbar sind und unter Umesterung und/oder Dehydratisierung umgesetzt werden können;

IV. einwertige Alkohole mit 1 bis 18 C-Atomen, z. B. Methanol, Ethanol, Isopropanol, Cyclohexanol, Benzylalkohol, Isodecanol, Nonanol, Octanol, Oleylalkohol.

Die Polyester können auf an sich bekannte Weise durch Kondensation in einer Inertgasatmosphäre bei Temperaturen von 100 bis 260 °C, vorzugsweise 130 bis 220 °C, in der Schmelze oder in azeotroper Fahrweise gewonnen werden, wie es z. B. in Methoden der Organischen Chemie (Houben-Weyl), Bd. 14/2, 1-5, 21-23, 40-44, Georg Thieme Verlag, Stuttgart, 1963 oder bei C. R. Martens, Alkyd Resins, 51-59, Reinhold Plastics Appl. Series, Reinhold Publishing Comp., New York, 1961, beschrieben ist.

Bevorzugte Acrylatharze, welche als OH-Komponente verwendet werden können, sind Homo- oder Copolymerisate, wobei z. B. folgende Monomere als Ausgangsprodukte gewählt werden können:

Ester der Acrylsäure und Methacrylsäure mit zweiwertigen, gesättigten, aliphatischen Alkoholen mit 2 bis 4 C-Atomen, wie z. B. 2-Hydroxyethylacrylat, 2-Hydroxypropylacrylat, 4-Hydroxybutylacrylat und die entsprechenden Methacrylsäureester; Acrylsäure und Methacrylsäurealkylester mit 1 bis 18, vorzugweise 1 bis 8 C-Atomen in der Alkoholkomponente, wie z. B. Methacrylat, Ethylacrylat, Propylacrylat, Isopropylacrylat, n-Butylacrylat, tert.-Butylacrylat, 2-Ethylhexylacrylat, Stearylacrylat und die entsprechenden Methacrylsäureester; Acrylsäure- und Methacrylsäurecyclohexylester; Acrylnitril und Methacrylnitril, Acrylamid und Methacrylamid; N-Methoxymethyl(meth)acrylsäureamid.

Besonders bevorzugte Acrylharze sind Copolymere aus

a. 0 bis 50 Gew.-% der Acryl- oder Methacrylsäure mit zwei- oder mehrwertigen Alkoholen, wie Butandiol-(1.4)-monoacrylat, Hydroxypropyl(meth)acrylat; ferner Vinylglykol, Vinylthioethanol, Allylalkohol, Butandiol-1.4-monovinylether;

b. 5 bis 95 Gew.-% Ester der Acrylsäure oder Methacrylsäure mit einwertigen Alkoholen, die 1 bis 12 Kohlenstoffatome enthalten, wie z. B. Methylmethacrylat, Ethylacrylat, n-Butylacrylat oder 2-Ethylhexylacrylat;

c. 0 bis 50 Gew.-% aromatische Vinylverbindungen, wie Styrol, Methylstyrol oder Vinyltoluol;

d. 0 bis 20 Gew.-% andere Monomere mit funktionellen Gruppen, wie z. B. Acrylsäure, Methacrylsäure, Crotonsäure, Itaconsäure, Maleinsäure, Fumarsäure, Maleinsäureanhydrid, Maleinsäurehalbester, Acrylamid, Methacrylamid, Acrylnitril oder N-Methylol(meth)acrylamid sowie Glycidyl(meth)acrylat, wobei der Anteil der Gruppe a. und/oder d. mindestens 5 Gew.-% beträgt.

Die Acrylatharze können nach den üblichen Methoden hergestellt werden, also durch Lösungs-, Suspensions-, Emulsions- oder Fällungspolymerisation; bevorzugt aber durch Substanzpolymerisation, die ihrerseits mittels UV-Licht initiiert werden kann.

Als weitere Polymerisationsinitiatoren werden die üblichen Peroxide oder Azoverbindungen, wie z. B. Dibenzoylperoxid, tert.-Butylperbenzoat oder Azodiisobutyronitril verwendet. Das Molekulargewicht kann z. B. mit Schwefelverbindungen, wie tert.-Dodecylmercaptan, geregelt werden.

Bevorzugte Polyether können z. B. durch Polyaddition von Epoxiden, wie Ethylenoxid, Propylenoxid, Butylenoxid, Trimethylenoxid, 3,3-Bis-(chlor-methyl)-oxacyclobutan, Tetrahydrofuran, Styroloxid, dem Bis-(2.5)-epoxypropylether des Diphenylolpropans oder Epichlorhydrin mit sich selbst, z. B. in Gegenwart von $BF_3$, oder durch Anlagerung dieser Epoxide, gegebenenfalls im Gemisch oder nacheinander, an Startkomponenten mit reaktionsfähigen Wasserstoffatomen, wie Alkohole oder Amine, z. B. Wasser, Ethylenglykol, Propylenglykol-(1.3) oder -(1.2), Pentamethylenglykol, Hexandiol, Decamethylenglykol, Trimethylolpropan, 4.4'-Dihydroxydiphenylpropan, Anilin, Ammoniak, Ethanolamin, Ethylendiamin, Di($\beta$-hydroxypropyl)-methylamin, Di-($\beta$-hydroxyethyl)-anilin, Hydrazin sowie auch hydroxyalkylierten Phenolen, wie z. B. 0.0-Di-($\beta$-hydroxyethyl)-resorcin, hergestellt werden.

Ebenso können hyxdroxylgruppenhaltige Polyurethane und/oder Polyharnstoffe eingesetzt werden.

Als Polyhydroxylverbindungen können selbstverständlich Gemische mehrerer Stoffe eingesetzt werden.

Das Mischungsverhältnis von hydroxylgruppenhaltigen Polymeren und Isocyanatkomponente wird in der Regel so gewählt, daß auf eine OH-Gruppe 0,6 bis 1,2, bevorzugt 0,8 bis 1,1, ganz besonders bevorzugt 1,0, blockierte NCO-Gruppen kommen.

Die Isocyanatkomponente wird für die Herstellung von PUR-Pulverlacken mit dem geeigneten hydroxylgruppenhaltigen Polymeren und gegebenenfalls Katalysatoren, wie Pigmenten, Füllstoffen und Verlaufmitteln, z. B. Siliconöl, flüssigen Acrylatharzen, gemischt und in der Schmelze homogenisiert. Dies kann in geeigneten Aggregaten, wie z. B. beheizbaren Knetern, vorzugsweise jedoch durch Extrudieren, erfolgen, wobei Temperaturobergrenzen von 130 bis 140 °C nicht überschritten werden sollten. Die extrudierte Masse wird nach Abkühlen auf Raumtemperatur und nach geeigneter Zerkleinerung zum sprühfertigen Pulver vermahlen. Das Auftragen des sprühfertigen Pulvers auf geeignete Substrate kann nach den bekannten Verfahren, wie z. B. durch elektrostatisches Pulversprühen, Wirbelsintern, elektrostatisches Wirbelsintern, erfolgen. Nach dem Pulverauftrag werden die beschichteten Werkstücke zur Aushärtung 60 bis 10 Minuten auf eine Temperatur von 160 bis 220 °C, vorzugsweise 30 bis 10 Minuten bei 180 bis 210 °C, erhitzt.

Die Isocyanatkomponente kann zur Herstellung lösemittelhaltiger PUR-Einkomponenten-Einbrennlacke in hierfür geeigneten Lösemitteln gelöst und mit dem für diesen Anwendungsbereich geeigneten hydroxylgruppenhaltigen Polyestern homogenisiert werden und in bekannter Weise mit den vorgenannten Zuschlagstoffen formuliert werden. Für die erfindungsgemäßen Einkomponenten-Einbrennlacke geeignete Lösemittel sind solche, deren unterer Siedepunkt bei etwa 100 °C liegt. Die obere Grenze des Siedepunktes des Lösemittels ist von den jeweiligen Einbrenntemperaturen abhängig. Wenn man bei höheren Temperaturen einbrennt, müssen die Siedepunkte der zu verwendenden Lösemittel bei höheren Temperaturen liegen. Als Lösemittel kommen unter anderem folgende infrage:

Kohlenwasserstoffe, wie z. B. Toluol, Xylol, SOLVESSO[R] 100, 150 und 200 (Aromatengemisch der Firma Esso), Tetralin, Decalin, Ester, wie z. B. Essigsäurebutylester und -hexylester, Ethylenglykolacetat, Butylglykolacetat, Methoxypropylacetat (MOP-A) usw., Ketone, wie z. B. Methylisobutylketon, Diisobutylketon, Isophoron. Die genannten Lösemittel können auch als Gemische eingesetzt werden.

Die PUR-Einkomponenten-Einbrennlacke eignen sich insbesondere für die Applikation auf Metalloberflächen, aber auch auf Gegenständen aus anderen Materialien, wie Glas oder Kunststoff. Die erfindungsgemäßen Lacke finden auch Anwendung in der Coil-Coating-Lackierung für witterungsbeständige Ein- und Zweischichtlackierungen. Der Auftrag der lösemittelhaltigen, gegebenenfalls pigmentierten Lacksysteme erfolgt durch Rakeln, Rollen, Spritzen, Gießen etc.. Die Härtung der erfindungsgemäßen Einkomponenten-PUR-Einbrennlacke erfolgt - je nach Anwendung - in einem Temperaturbereich von 160 bis 350 °C, vorzugsweise zwischen 180 bis 300 °C, in einer Zeit von 30 Minuten bis 30 Sekunden.

Die Lackfilme weisen hervorragende lacktechnische Eigenschaften, insbesondere Flexibilität und Witterungsbeständigkeit, auf.

## A Herstellung der als Katalysatoren eingesetzten N-(Hydroxyalkyl)-ammoniumsalze

### Allgemeine Herstellungsvorschrift

In einem mit Rührer, Rückflußkühler und Tropftrichter versehenen 1 l-Dreihalskolben werden 1 mol tert. Amin mit 1 mol Monocarbonsäure a) lösungsmittelfrei und b) in 50 %iger Dipropylenglykol-Lösung neutralisiert. In diese Mischung werden dann unter intensiver Rührung bei Raumtemperatur 1 mol eines Monoepoxids, z. B. Propylenoxid, innerhalb 2 h zugegeben. Anschließend wird das Reaktionsgemisch 24 h bei Raumtemperatur stehengelassen. Zur Entfernung von nicht umgesetzten flüchtigen Bestandteilen (z. B. Propylenoxid) wird das Reaktionsgemisch noch 6 h bei 45 bis 50 °C im Wasserstrahlvakuum erhitzt.

Unter Anwendung dieser Verfahrensweise wurden die in der nachfolgenden Tabelle zusammengestellten Verbindungen hergestellt.

| Beisp. | Gew.-Teile | | | |
|---|---|---|---|---|
| | Tertiäres Amin | Monocarbonsäure | Monoepoxid | Lösemittel |
| A 1 | 20,8 Trimethylamin | 21,2 Essigsäure | 18,0 Propylenoxid | 40,0 DPG |
| A 2 | 23,4 Trimethylamin | 18,2 Ameisensäure | 20,6 Propylenoxid | 37,8 DPG |
| A 3 | 13,6 Trimethylamin | 33,2 2-Ethylhexansäure | 12,2 Propylenoxid | 41,0 DPG |
| A 4 | 26,6 DABCO* T | 53,3 2-Ethylhexansäure | 20,1 Propylenoxid | - |
| A 5 | 29,7 DABCO T | 48,0 Hexansäure | 22,3 Propylenoxid | - |
| A 6 | 20,6 DABCO T | 33,4 Hexansäure | 15,5 Propylenoxid | 30,5 DPG |
| A 7 | 16,6 DABCO T | 26,4 Hexansäure | 29,6 Butylglycidether | 27,4 DEG |
| A 8 | 15,8 Pentamethyldiethylentriamin | 26,4 2-Ethylhexansäure | 21,1 Hexandioldiglycidether | 36,7 DEG |

DABCO T: N.N.N'-Trimethyl-N'-($\beta$-hydroxyethyl)-ethylendiamin

EG: Ethylenglykol DEG: Diethylenglykol DPG: Dipropylenglykol

## B Durchführung des erfindungsgemäßen Verfahrens

### Beispiel 1

2 000 Gew.-T. IPDI und 6,5 Gew.-T. Katalysator A 3 wurden unter intensiver Rührung auf 70 °C erhitzt. Bei dieser Temperatur setzte die Reaktion ein. Durch die freiwerdende Reaktionswärme stieg die Temperatur des Reaktionsgemisches innerhalb ca. 5 min auf 142 °C. Mit dem Erreichen des Temperaturmaximums war die Reaktion beendet. Der NCO-Gehalt der Reaktionsmischung betrug 26,1 %. Das Reaktionsgemisch wurde nun auf 120 °C abgekühlt und mit 5 Gew.-T. $H_3PO_4$ versetzt. Anschließend wurden 1 400 Gew.-T. $\epsilon$-Caprolactam portionsweise so zugegeben, daß die Temperatur des Reaktionsgemisches nicht über 140 °C stieg. Nach beendeter $\epsilon$-Caprolactamzugabe wurde zur Vervollständigung der Reaktion noch 2 h bei 140 °C weiter erhitzt.

Das Reaktionsprodukt hatte einen Gehalt an freiem NCO von 0,6 %, einen Schmelzbereich von 82 - 92 °C und einen Glasumwandlungspunkt (DSC) von 45 - 63 °C.

### Beispiel 2

500 Gew.-T. IPDI und 1,5 Gew.-T. Katalysator A 5 wurden unter intensiver Rührung auf 55 °C aufgeheizt. Hier setzte die Reaktion ein. Durch Zugabe von IPDI wurde die Umsetzung so geregelt, daß die Temperatur des Reaktionsgemisches innerhalb 1,5 h auf 78 °C stieg. Für diese Temperatursteigerung wurden 500 Gew.-T. IPDI eingesetzt. Der NCO-Gehalt betrug 26,5 %. Das Reaktionsgemisch wurde dann auf 120 °C erhitzt, wobei der NCO-Gehalt auf 26 % sank. Nach Zugabe von 1,2 Gew.-T. $H_3PO_4$ wurde das Reaktionsgemisch mit 700 Gew.-T. $\epsilon$-Caprolactam entsprechend Beispiel 1 umgesetzt. Das Reaktionsprodukt hatte einen Gehalt an freiem NCO von 0,4 %; der Schmelzbereich betrug 82 - 90 °C, der Glasumwandlungspunkt 44 - 63 °C.

### Beispiel 3

1 000 Gew.-T. IPDI wurden bei Raumtemperatur unter intensiver Rührung mit 5 Gew.-T. Katalysator A 1 gemischt. Innerhalb von ca. 10 min stieg die Temperatur des Reaktionsgemisches auf 149 °C. Der NCO-Gehalt des Reaktionsgemisches betrug 20,5 %. Nachdem das Reaktionsgemisch auf 120 °C abgekühlt und mit 5 Gew.-T. $H_3PO_4$ versetzt wurde, erfolgte die $\epsilon$-Caprolactam-Blockierung (828 Gew.-T. $\epsilon$-Caprolactam) entsprechend Beispiel 1.

Das Reaktionsprodukt enthielt kein freies NCO; der Schmelzbereich betrug 74 - 85 °C; der Glasumwandlungspunkt (DSC) 35 - 49 °C.

**C Polyolkomponente**

Allgemeine Herstellungsvorschrift

Die Ausgangskomponenten - Terephthalsäure (Ts) und/oder Isophthalsäure (Is) und/oder Caprolacton (Cl) und/oder Dimethylterephthalat (DMT), Hexandiol-1.6 (HD) und/oder Neopentylglykol (NPG) und/oder 1,4-Dimethylolcyclohexan (DMC) und/oder 2.2.4(2.4.4)-Trimethylhexandiol (TMH-d.) und Trimethylolpropan (TMP) - werden in einen Reaktor gegeben und mit Hilfe eines Ölbades erwärmt. Nachdem die Stoffe zum größten Teil aufgeschmolzen sind, werden bei einer Temperatur von 160 °C 0,05 Gew.-% Di-n-butylzinnoxid als Katalysator zugesetzt. Die erste Methanolabspaltung tritt bei einer Temperatur von ca. 170 °C auf. Innerhalb 6 bis 8 h wird die Temperatur auf 200 bis 230 °C erhöht und innerhalb weiterer 12 bis 15 h die Reaktion zu Ende geführt. Der Polyester wird auf 200 °C abgekühlt und durch Anlegen von Vakuum (1,33 mbar) innerhalb 30 bis 45 min weitgehend von flüchtigen Anteilen befreit. Während der gesamten Reaktionszeit wird das Sumpfprodukt gerührt und ein schwacher $N_2$-Strom durch das Reaktionsgemisch geleitet.

Folgende Tabelle gibt die Polyesterzusammensetzungen und die entsprechenden physikalischen und chemischen Kenndaten wieder.

## Polyester

| Beisp. C | Ausgangskomponenten | | | | | | Chemische und physik. Kenndaten | | | | |
|---|---|---|---|---|---|---|---|---|---|---|---|
| | Ts Mol | DMT Mol | HD Mol | NPG Mol | DMC Mol | TMP Mol | OH-Zahl mg KOH/g | Säurezahl mg KOH/g | Schmelzp. °C | DTA* °C | Visk.b. 160 mPa·s |
| 1 | 10 | 10 | 6,25 | 10,5 | 2 | 2,9 | 55-60 | 3-4 | 75 | 50 | 25 000 |
| 2 | 12 | 12 | 6,75 | 11,5 | 5 | 3 | 58-63 | 3-5 | 80 | 50 | 19 000 |
| 3 | Polyester der Hoechst AG ALFTALAT AN 739 | | | | | | 55-60 | 2-3 | 70 | 50 | 28 000 |
| | Is | Cl | | TMH-d | | | | | | | |
| 4 | 7 | - | 5 | 1 | - | 2 | 105-112 | < 2 | - | (-3)-(+9) | - |
| 5 | 10 | - | 7,5 | - | - | 4 | 135-141 | < 2 | - | - | - |
| 6 | 7 | 1 | 6 | - | - | 2 | 91-95 | < 2 | - | (-8)-(+6) | 2 260 |

\* Glasumwandlungstemperatur
Polyester 1-3 für PUR-Pulverlacke
Polyester 4-7 für lösemittelhaltige PUR-Einkomponenten-Einbrennlacke

## D Polyurethan-Pulverlacke

Allgemeine Herstellungsvorschrift

Die gemahlenen Produkte, Vernetzer, Polyester, Verlaufmittel-V-Masterbatch, werden gegebenenfalls mit dem Weißpigment in einem Kollergang innig vermischt und anschließend im Extruder bei 80 bis 110 °C homogenisiert. Nach dem Erkalten wird das Extrudat gebrochen und mit einer Stiftmühle auf eine Korngröße < 100 μm gemahlen. Das so hergestellte Pulver wird mit einer elektrostatischen Pulverspritzanlage bei 60 KV auf entfettete, gegebenenfalls vorbehandelte Eisenbleche appliziert und in einem Umlufttrokkenschrank bei Temperaturen zwischen 180 und 200 °C eingebrannt.

Verlaufmittel-Masterbatch

Es werden 10 Gewichtsprozent des Verlaufmittels - ein handelsübliches Copolymeres von Butylacrylat und 2-Ethylhexylacrylat - in dem entsprechenden Polyester in der Schmelze homogenisiert und nach dem Erstarren zerkleinert.

Die Abkürzungen in den folgenden Tabellen bedeuten:

SD = Schichtdicke in $\mu$m
HK = Härte nach König (sec) (DIN 53 157)
HB = Härte nach Buchholz (DIN 53 153)
ET = Tiefung nach Erichsen (mm) (DIN 53 156)
GS = Gitterschnittprüfung (DIN 53 151)
GG 60 ° ⌡ = Messung des Glanzes nach Gardner (ASTM-D 523)
Imp. rev. = Impact reverse in g•m
T-Bend-Test = gemessen an 0,8 mm starken lackierten Eisenblechen. Hierbei wird das Blech mit der Lackseite nach außen in verschiedenen Krümmungsradien um 180 ° gebogen, wobei sich die Krümmungsradien dadurch ergeben, daß man entweder keine (Meßzahl 0) oder mehrere (Meßzahl > 0) unlackierte Bleche gleicher Stärke als Abstandshalter bei der Biegung verwendet. Die Meßzahl gibt an, unter welchen Bedingungen die Biegung eben noch erfolgen kann, ohne daß bei 10facher Vergrößerung Risse erkennbar sind. (Prüfnorm der European Coil Coating Association).

Anwendungstechnisches Beispiel 1

**Pigmentierter Lack**

Nach dem beschriebenen Verfahren wurde der Pulverlack mit folgender Rezeptur hergestellt, appliziert und zwischen 180 und 200 °C eingebrannt.

411,3 Gew.-T. Polyester gemäß Beispiel C 3
138,7 Gew.-T. Vernetzer gemäß Beispiel B 1
400,0 Gew.-T. Weißpigment (TiO$_2$)
50,0 Gew.-T. Verlaufmittel-Masterbatch

| Einbrenn-bedingungen | Mechanische Kenndaten | | | | | | |
|---|---|---|---|---|---|---|---|
| Zeit/Temp. | SD | HK | HB | ET | GS | Imp.rev. | GG 60 °⌡ |
| 10'/200 °C | 70-80 | 190 | 125 | 9,2-9,5 | 0 | 460,8 | 84 |
| 15'/200 °C | 70-80 | 189 | 125 | > 10 | 0 | 691,2 | 84 |
| 20'/200 °C | 65-70 | 191 | 111 | > 10 | 0 | > 944,6 | 83 |
| 15'/180 °C | 65-70 | 195 | 125 | 8,0-8,5 | 0 | 230,4 | 84 |
| 20'/180 °C | 60-80 | 191 | 125 | 9,8->10 | 0 | 345,6 | 84 |
| 25'/180 °C | 60-70 | 193 | 125 | > 10 | 0 | 806,4 | 83 |

Anwendungstechnisches Beispiel 2

**Pigmentierter Lack**

Nach dem beschriebenen Verfahren wurde der Pulverlack mit folgender Rezeptur hergestellt, appliziert und zwischen 180 und 200 °C eingebrannt.

412,7 Gew.-T. Polyester gemäß Beispiel C 3
137,3 Gew.-T. Vernetzer gemäß Beispiel B 2
400,0 Gew.-T. Weißpigment (TiO$_2$)
50,0 Gew.-T. Verlaufmittel-Masterbatch

| Einbrenn-bedingungen | Mechanische Kenndaten | | | | | | |
|---|---|---|---|---|---|---|---|
| Zeit/Temp. | SD | HK | HB | ET | GS | Imp.rev. | GG 60 °⟩ |
| 10'/200 °C | 60 | 194 | 111 | > 10 | 0 | > 944,6 | 86 |
| 15'/200 °C | 50-60 | 187 | 111 | > 10 | 0 | > 944,6 | 86 |
| 20'/200 °C | 50-60 | 188 | 111 | > 10 | 0 | > 944,6 | 86 |
| 15'/180 °C | 50-60 | 195 | 111 | > 10 | 0 | > 944,6 | 86 |
| 20'/180 °C | 55-60 | 190 | 111 | > 10 | 0 | > 944,6 | 87 |
| 25'/180 °C | 50-60 | 188 | 111 | > 10 | 0 | > 944,6 | 86 |

Anwendungstechnisches Beispiel 3

**Pigmentierter Lack**

Nach dem beschriebenen Verfahren wurde der Pulverlack mit folgender Rezeptur hergestellt, appliziert und zwischen 180 und 200 °C eingebrannt.

412,7 Gew.-T. Polyester gemäß Beispiel C 3
137,3 Gew.-T. Vernetzer gemäß Beispiel B 4
400,0 Gew.-T. Weißpigment (TiO$_2$)
50,0 Gew.-T. Verlaufmittel-Masterbatch

EP 0 512 213 B1

| Einbrenn-bedingungen | Mechanische Kenndaten | | | | | | |
|---|---|---|---|---|---|---|---|
| Zeit/Temp. | SD | HK | HB | ET | GS | Imp.rev. | GG 60 °⌥ |
| 10'/200 °C | 50-70 | 191 | 111 | > 10 | 0 | 806,4 | 86 |
| 15'/200 °C | 60 | 194 | 111 | > 10 | 0 | 806,4 | 87 |
| 20'/200 °C | 60-70 | 193 | 125 | > 10 | 0 | > 944,6 | 86 |
| 15'/180 °C | 50-60 | 190 | 111 | > 10 | 0 | 806,4 | 85 |
| 20'/180 °C | 50-60 | 195 | 125 | > 10 | 0 | > 944,6 | 86 |
| 25'/180 °C | 60 | 189 | 111 | > 10 | 0 | > 944,6 | 86 |

Anwendungstechnisches Beispiel 4

**Pigmentierter Lack**

Nach dem beschriebenen Verfahren wurde der Pulverlack mit folgender Rezeptur hergestellt, appliziert und zwischen 180 und 200 °C eingebrannt.

410,5 Gew.-T. Polyester gemäß Beispiel C 3
139,5 Gew.-T. Vernetzer gemäß Beispiel B 5
400,0 Gew.-T. Weißpigment ($TiO_2$)
50,0 Gew.-T. Verlaufmittel-Masterbatch

| Einbrenn-bedingungen | Mechanische Kenndaten | | | | | | |
|---|---|---|---|---|---|---|---|
| Zeit/Temp. | SD | HK | HB | ET | GS | Imp.rev. | GG 60 °⌥ |
| 10'/200 °C | 60-70 | 197 | 111 | > 10 | 0 | 691,2 | 85 |
| 15'/200 °C | 50-60 | 194 | 111 | > 10 | 0 | 806,4 | 84 |
| 20'/200 °C | 60 | 196 | 111 | > 10 | 0 | > 944,6 | 86 |
| 15'/180 °C | 60 | 193 | 111 | > 10 | 0 | 806,4 | 86 |
| 20'/180 °C | 60-70 | 198 | 125 | > 10 | 0 | 806,4 | 85 |
| 25'/180 °C | 50-70 | 191 | 111 | > 10 | 0 | > 944,6 | 85 |

11

Anwendungstechnisches Beispiel 5

**Pigmentierter Lack**

Nach dem beschriebenen Verfahren wurde der Pulverlack mit folgender Rezeptur hergestellt, appliziert und zwischen 180 und 200 °C eingebrannt.

412,7 Gew.-T. Polyester gemäß Beispiel C 3
137,3 Gew.-T. Vernetzer gemäß Beispiel B 6
400,0 Gew.-T. Weißpigment (TiO$_2$)
50,0 Gew.-T. Verlaufmittel-Masterbatch

| Einbrenn-bedingungen | Mechanische Kenndaten | | | | | | |
|---|---|---|---|---|---|---|---|
| Zeit/Temp. | SD | HK | HB | ET | GS | Imp.rev. | GG 60 °} |
| 10'/200 °C | 50-60 | 197 | 111 | > 10 | 0 | > 944,6 | 87 |
| 15'/200 °C | 50-55 | 191 | 125 | > 10 | 0 | > 944,6 | 86 |
| 20'/200 °C | 50-60 | 192 | 111 | > 10 | 0 | > 944,6 | 86 |
| 15'/180 °C | 50-70 | 202 | 125 | > 10 | 0 | 115,2 | 87 |
| 20'/180 °C | 60-70 | 195 | 125 | > 10 | 0 | 691,2 | 87 |
| 25'/180 °C | 50-60 | 190 | 111 | > 10 | 0 | > 944,6 | 86 |

Anwendungstechnisches Beispiel 6

**Pigmentierter Lack**

Nach dem beschriebenen Verfahren wurde der Pulverlack mit folgender Rezeptur hergestellt, appliziert und zwischen 180 und 200 °C eingebrannt.

423,5 Gew.-T. Polyester gemäß Beispiel C 3
126,5 Gew.-T. Vernetzer gemäß Beispiel B 6
400,0 Gew.-T. Weißpigment (TiO$_2$)
50,0 Gew.-T. Verlaufmittel-Masterbatch

EP 0 512 213 B1

| Einbrenn-bedingungen | Mechanische Kenndaten | | | | | | |
|---|---|---|---|---|---|---|---|
| Zeit/Temp. | SD | HK | HB | ET | GS | Imp.rev. | GG 60 °% |
| 10'/200 °C | 70-80 | 197 | 111 | > 10 | 0 | 460,8 | 87 |
| 15'/200 °C | 60-70 | 189 | 111 | > 10 | 0 | > 944,6 | 87 |
| 20'/200 °C | 50-70 | 190 | 100 | > 10 | 0 | > 944,6 | 86 |
| 15'/180 °C | 60-70 | 197 | 100 | > 10 | 0 | 460,8 | 88 |
| 20'/180 °C | 60-65 | 194 | 100 | > 10 | 0 | > 944,6 | 89 |
| 25'/180 °C | 60 | 192 | 100 | > 10 | 0 | > 944,6 | 88 |

Anwendungstechnisches Beispiel 7

**Pigmentierter Lack**

Nach dem beschriebenen Verfahren wurde der Pulverlack mit folgender Rezeptur hergestellt, appliziert und zwischen 180 und 200 °C eingebrannt.

407,5 Gew.-T. Polyester gemäß Beispiel C 3
142,5 Gew.-T. Vernetzer gemäß Beispiel B 6
400,0 Gew.-T. Weißpigment ($TiO_2$)
50,0 Gew.-T. Verlaufmittel-Masterbatch

| Einbrenn-bedingungen | Mechanische Kenndaten | | | | | | |
|---|---|---|---|---|---|---|---|
| Zeit/Temp. | SD | HK | HB | ET | GS | Imp.rev. | GG 60 °% |
| 10'/200 °C | 60 | 195 | 125 | > 10 | 0 | 230,4 | 85 |
| 15'/200 °C | 70 | 188 | 125 | > 10 | 0 | > 691,2 | 86 |
| 20'/200 °C | 60-70 | 190 | 125 | > 10 | 0 | > 944,6 | 85 |
| 15'/180 °C | 60-70 | 196 | 125 | 9,0->10 | 0 | 230,4 | 87 |
| 20'/180 °C | 75-80 | 195 | 125 | 9,8->10 | 0 | 460,8 | 87 |
| 25'/180 °C | 60-75 | 201 | 111 | 9,8->10 | 0 | 806,4 | 86 |

13

Anwendungstechnisches Beispiel 8

**Pigmentierter Lack**

Nach dem beschriebenen Verfahren wurde der Pulverlack mit folgender Rezeptur hergestellt, appliziert und zwischen 180 und 200 °C eingebrannt.

402,4 Gew.-T. Polyester gemäß Beispiel C 1
147,6 Gew.-T. Vernetzer gemäß Beispiel B 6
400,0 Gew.-T. Weißpigment ($TiO_2$)
50,0 Gew.-T. Verlaufmittel-Masterbatch

| Einbrenn-bedingungen | Mechanische Kenndaten | | | | | | |
|---|---|---|---|---|---|---|---|
| Zeit/Temp. | SD | HK | HB | ET | GS | Imp.rev. | GG 60 °} |
| 10'/200 °C | 60-70 | 195 | 111 | > 10 | 0 | 576 | 86 |
| 15'/200 °C | 70 | 197 | 111 | > 10 | 0 | 806,4 | 87 |
| 20'/200 °C | 50-60 | 194 | 125 | > 10 | 0 | > 944,6 | 87 |
| 15'/180 °C | 60-70 | 194 | 125 | > 10 | 0 | 806,4 | 86 |
| 20'/180 °C | 60-75 | 196 | 111 | > 10 | 0 | > 944,6 | 85 |
| 25'/180 °C | 60-70 | 193 | 111 | > 10 | 0 | > 944,6 | 87 |

Anwendungstechnisches Beispiel 9

**Pigmentierter Lack**

Nach dem beschriebenen Verfahren wurde der Pulverlack mit folgender Rezeptur hergestellt, appliziert und zwischen 180 und 200 °C eingebrannt.

409,1 Gew.-T. Polyester gemäß Beispiel C 2
140,9 Gew.-T. Vernetzer gemäß Beispiel B 6
400,0 Gew.-T. Weißpigment ($TiO_2$)
50,0 Gew.-T. Verlaufmittel-Masterbatch

14

| Einbrenn-bedingungen | Mechanische Kenndaten | | | | | | |
|---|---|---|---|---|---|---|---|
| Zeit/Temp. | SD | HK | HB | ET | GS | Imp.rev. | GG 60 °⁀ |
| 10'/200 °C | 70-80 | 190 | 111 | > 10 | 0 | 691,2 | 86 |
| 15'/200 °C | 60-70 | 186 | 111 | > 10 | 0 | > 944,6 | 86 |
| 20'/200 °C | 70 | 189 | 111 | > 10 | 0 | > 944,6 | 86 |
| 15'/180 °C | 60-80 | 188 | 111 | > 10 | 0 | 576 | 86 |
| 20'/180 °C | 70-80 | 196 | 111 | > 10 | 0 | 806,4 | 87 |
| 25'/180 °C | 60-70 | 190 | 111 | > 10 | 0 | > 944,6 | 86 |

Anwendungstechnisches Beispiel 10

**Pigmentierter Lack**

Nach dem beschriebenen Verfahren wurde der Pulverlack mit folgender Rezeptur hergestellt, appliziert und zwischen 180 und 200 °C eingebrannt.

412,0 Gew.-T. Polyester gemäß Beispiel C 3
138,0 Gew.-T. Vernetzer gemäß Beispiel B 7
400,0 Gew.-T. Weißpigment (TiO$_2$)
50,0 Gew.-T. Verlaufmittel-Masterbatch

| Einbrenn-bedingungen | Mechanische Kenndaten | | | | | | |
|---|---|---|---|---|---|---|---|
| Zeit/Temp. | SD | HK | HB | ET | GS | Imp.rev. | GG 60 °⁀ |
| 10'/200 °C | 50-60 | 188 | 111 | > 10 | 0 | 860,4 | 87 |
| 15'/200 °C | 50-65 | 190 | 111 | > 10 | 0 | 806,4 | 86 |
| 20'/200 °C | 60-70 | 189 | 111 | > 10 | 0 | > 944,6 | 86 |
| 15'/180 °C | 50-60 | 186 | 125 | > 10 | 0 | 806,4 | 86 |
| 20'/180 °C | 50-55 | 189 | 125 | > 10 | 0 | > 944,6 | 88 |
| 25'/180 °C | 50-60 | 190 | 125 | > 10 | 0 | > 944,6 | 88 |

Anwendungstechnisches Beispiel 11

**Pigmentierter Lack**

Nach dem beschriebenen Verfahren wurde der Pulverlack mit folgender Rezeptur hergestellt, appliziert und zwischen 180 und 200 °C eingebrannt.

411,3 Gew.-T. Polyester gemäß Beispiel C 3
138,7 Gew.-T. Vernetzer gemäß Beispiel B 8
400,0 Gew.-T. Weißpigment ($TiO_2$)
50,0 Gew.-T. Verlaufmittel-Masterbatch

| Einbrenn-bedingungen | Mechanische Kenndaten | | | | | | |
|---|---|---|---|---|---|---|---|
| Zeit/Temp. | SD | HK | HB | ET | GS | Imp.rev. | GG 60 °$\}$ |
| 10'/200 °C | 60-70 | 190 | 111 | > 10 | 0 | 691,2 | 87 |
| 15'/200 °C | 50-65 | 187 | 125 | > 10 | 0 | 806,4 | 86 |
| 20'/200 °C | 50-60 | 189 | 111 | > 10 | 0 | > 944,6 | 86 |
| 15'/180 °C | 60-70 | 191 | 111 | > 10 | 0 | 806,4 | 86 |
| 20'/180 °C | 55-65 | 188 | 111 | > 10 | 0 | 806,4 | 87 |
| 25'/180 °C | 50-60 | 189 | 125 | > 10 | 0 | > 944,6 | 86 |

**E Polyurethan-Einkomponenten-Einbrennlacke**

1. Herstellung der Vernetzer-Lösung

600 Gew.-T. des gemäß Beispiel B 6 hergestellten $\epsilon$-Caprolactam-blockierten, isocyanuratgruppenhaltigen IPDI-Adduktes wurden in 400 Gew.-T. Lösemittelgemisch unter Rühren bei 80 - 90 °C gelöst und die Auslaufzeit im DIN-4-Becher bei 20 °C bestimmt.
a) n-Butylacetat/Xylol 3 : 5 = 49 Sekunden
b) MOP-A/SOLVESSO[R] 100 1 : 3 = 63 Sekunden

2. Herstellung der Polyester-Lösung

600 Gew.-T. Polyester gemäß Beispielen C 4 - C 6 wurden unter Rühren in 400 Gew.-T. Lösemittelgemisch bei 80 - 90 °C gelöst und die Auslaufzeit im DIN-4-Becher bei 20 °C bestimmt.
c) C 4 n-Butylacetat/Xylol 3 : 5 = 271 Sekunden
d) C 5 n-Butylacetat/Xylol 3 : 5 = 471 Sekunden
c) C 6 MOP-A/SOLVESSO[R] 100 1 : 3 = 855 Sekunden

3. Herstellung der PUR-Einkomponenten-Einbrennlacke

**a) Lackstammlösung**

Die 60 gew.-%ige Polyesterlösung wurde unter Rühren mit der berechneten Menge der 60 gew.-%igen Vernetzerlösung bei 60 - 80 °C homogenisiert und die Auslaufzeit im DIN-4-Becher bei 20 °C bestimmt.

16

60 Gew.-%ige Lackstammlösungen
a) gemäß Beispiel C 4 und gemaß Beispiel D 1a = 171 Sekunden
b) gemäß Beispiel C 5 und gemäß Beispiel D 1a = 236 Sekunden
c) gemäß Beispiel C 6 und gemäß Beispiel D 1b = 303 Sekunden

**b) Pigmentierte Lacklösung**

Die gemäß E 3 hergestelten Lackstammlösungen wurden, falls erforderlich, mit weiterem Lösemittelgemisch - entsprechend der Stammlösung - versetzt und anschließend mit Weißpigment ($TiO_2$) und einem in der PUR-Chemie üblichen Verlaufmittel und Entschäumer in der Kugelrührwerksmühle abgerieben.
Die Applikation dieser pigmentierten Lacke erfolgte auf entfetteten und/oder vorbehandelten 0,8 - 1 mm Stahl- und/oder Aluminiumblechen; die Aushärtung wurde in einem Labor-Umlufttrockenschrank durchgeführt; die Härtungstemperaturen lagen zwischen 180 und 250 °C; die Schichtdicke der Lackfilme lag je nach Anwendung zwischen 25 und 50 $\mu$m.

Anwendungstechnisches Beispiel 12

Die Herstellung und Applikation sowie Härtung des Lackes erfolgte gemäß E 3 b.

Rezeptur:

47,6 Gew.-% Polyesterlösung gemäß E 2 a
25,2 Gew.-% Vernetzerlösung gemäß E 1 a
26,0 Gew.-% Weißpigment ($TiO_2$)
1,1 Gew.-% Entschäumer (Byk-spezial)
0,1 Gew.-% Verlaufmittel (Siliconöl OL)

| Härtungs-bedingungen | Mechanische Kenndaten | | | | | |
|---|---|---|---|---|---|---|
| Zeit/Temp. | HK | HB | ET | Bleistifthärte | GS | GG 60 °⟩ |
| 3'/250 °C | 192 | 125 | 8,1 | 2 H | 0 | 86 |
| 10'/200 °C | 195 | 125 | 8,4 | 2 H | 0 | 87 |
| 15'/200 °C | 200 | 125 | 8,0 | 2 H | 0 | 87 |
| 15'/180 °C | 195 | 111 | 8,0 | 2 H | 0 | 88 |
| 20'/180 °C | 200 | 125 | 8,0 | 2 H | 0 | 89 |

Die Schichtdicke der Lackfilme betrug 30 bis 40 $\mu$m.

EP 0 512 213 B1

Anwendungstechnisches Beispiel 13

Die Herstellung und Applikation sowie Härtung des Lackes erfolgte gemäß E 3 b.

Rezeptur:

43,4 Gew.-% Polyesterlösung gemäß E 2 b
27,9 Gew.-% Vernetzerlösung gemäß E 1 a
2,1 Gew.-% Lösungsmittelgemisch gemäß der eingesetzten Lacklösung
25,5 Gew.-% Weißpigment ($TiO_2$)
1,0 Gew.-% Entschäumer (Byk-spezial)
0,1 Gew.-% Verlaufmittel (Siliconöl OL)

| Härtungs-bedingungen | Mechanische Kenndaten | | | | | |
|---|---|---|---|---|---|---|
| Zeit/Temp. | HK | HB | ET | Bleistifthärte | GS | GG 60 °} |
| 3'/250 °C | 196 | 125 | 8,0 | 2 H | 0 | 85 |
| 10'/200 °C | 195 | 125 | 8,1 | 2 H | 0 | 84 |
| 15'/200 °C | 201 | 125 | 8,1 | 2 H | 0 | 85 |
| 15'/180 °C | 201 | 125 | 8,3 | 2 H | 0 | 83 |
| 20'/180 °C | 204 | 125 | 8,1 | 2 H | 0 | 84 |

Die Schichtdicke der Lackfilme betrug 30 bis 45 $\mu$m.

Anwendungstechnisches Beispiel 14

Die Herstellung und Applikation sowie Hartung des Lackes erfolgte gemäß E 3 b.

Rezeptur:

42,8 Gew.-% Polyesterlösung gemäß E 2 c
19,7 Gew.-% Vernetzerlösung gemäß E 1 b
7,0 Gew.-% Lösungsmittelgemisch gemäß der eingesetzten Lacklösung
29,4 Gew.-% Weißpigment ($TiO_2$)
1,0 Gew.-% Entschäumer (Byk-spezial)
0,1 Gew.-% Verlaufmittel (Siliconöl OL)

18

| Härtungs-bedingungen | Mechanische Kenndaten | | | | | | | |
|---|---|---|---|---|---|---|---|---|
| Zeit/Temp. | HK | HB | ET | Impact reverse | T-Bend-Test | Bleistift-härte | GS | GG 60 °} |
| 3'/250 °C | 181 | 125 | > 10 | >944,6 | 0 | 2 H | 0 | 84 |
| 7'/200 °C | 175 | 125 | > 10 | >944,6 | 0 | 2 H | 0 | 83 |
| 10'/200 °C | 180 | 125 | > 10 | >944,6 | 0 | 2 H | 0 | 84 |
| 12'/180 °C | 170 | 125 | > 10 | >944,6 | 0 | 2 H | 0 | 83 |
| 15'/180 °C | 174 | 125 | > 10 | >944,6 | 0-1 | 2 H | 0 | 86 |

Die Schichtdicke der Lackfilme betrug 25 bis 30 $\mu$m.

**Patentansprüche**

1. Verfahren zur Herstellung eines Lackpolyisocyanats auf Basis eines $\epsilon$-Caprolactam blockierten, Isocyanuratgruppen enthaltenden Isophorondiisocyanats, das in an sich bekannter Weise durch Trimerisierung des Isophorondiisocyanats mit quaternären N-(Hydroxyalkyl)-ammoniumsalzen von Carbonsäuren und anschließende $\epsilon$-Caprolactam-Blockierung des Isocyanatoisocyanurat-Gemisches hergestellt wird, dadurch gekennzeichnet,
   daß vor der $\epsilon$-Caprolactam-Blockierung das Isocyanatoisocyanurat-Gemisch des IPDI mit 0,1 - 1 Gew.-% Phosphor- bzw. phosphoriger Säure versetzt wird und pro NCO-Äquivalent des Isocyanatoisocyanurat-Gemisches 0,7 - 1 Mol $\epsilon$-Caprolactam zur Reaktion gelangen.

**Claims**

1. Method of producing a polyisocyanate varnish based on an $\epsilon$-caprolactam blocked isophorondiisocyanate which contains isocyanurate groups and is produced in a manner which is known per se by trimerisation of the isophorondiisocyanate with quaternary N-(hydroxyalkyl)-ammonium salts of carboxylic acids and subsequent $\epsilon$-caprolactam blocking of the isocyanatoisocyanurate mixture, characterised in that before the $\epsilon$-caprolactam blocking, the isocyanatoisocyanurate mixture of the IPDI is reacted with 0.1 - 1 % by weight of phosphoric or phosphorous acid and 0.7 - 1 mol of $\epsilon$-caprolactam is brought to reaction per NCO equivalent of the isocyanatoisocyanurate mixture.

**Revendications**

1. Procédé de production d'un polyisocyanate pour laque à base d'un isophorone diisocyanate contenant des groupes isocyanurate, bloqué par l'$\epsilon$-caprolactame, qui est produit d'une manière connue en soi par trimérisation de l'isophorone diisocyanate à l'aide de sels d'ammonium quaternaires N-(hydroxyalcoyl) d'acides carboxyliques et ensuite bloquage par l'$\epsilon$-caprolactame du mélange d'isocyanato/isocyanurate, caractérisé en ce qu'avant le bloquage par l'$\epsilon$-caprolactame, le mélange d'isocyanato/isocyanurate de l'IPDI est déplacé par 0,1 à 1 % en poids d'acide phosphorique ou d'acide phosphoreux et en ce que par équivalent de NCO du mélange isocyanato/isocyanurate 0,7 à 1 mol d'$\epsilon$-caprolactame vient en réaction.